# EUROPÄISCHE PATENTSCHRIFT

(11) **EP 0 442 256 B1**
(45) Veröffentlichungstag und Bekanntmachung des Hinweises auf die Patenterteilung: **29.05.1996**
(21) Anmeldenummer: 91100184.0
(22) Anmeldetag: 08.01.1991
(51) Int. Cl.: A61F 2/36, A61F 2/38, A61L 27/00, B29C 70/00

(54) **Knochenimplantat**
Bone implant
Implant osseux

(30) Priorität: 14.02.1990 DE 4004472; 14.02.1990 DE 4004475
(43) Veröffentlichungstag der Anmeldung: 21.08.1991
(73) Patentinhaber: MAN Ceramics GmbH, 94453 Deggendorf (DE)
(72) Erfinder: Wenner, Ulrich, Dipl.-Ing., W-3110 Uelzen 1 (DE); Dierl, Rudolf, W-8060 Dachau (DE)

(56) Entgegenhaltungen:
- EP-A- 0 225 838
- EP-A- 0 245 846
- EP-A- 0 311 749
- DE-A- 2 753 568
- DE-A- 3 536 894
- DE-A- 3 909 545

## Beschreibung

Die Erfindung bezieht sich auf ein Knochenimplantat, enthaltend einen Grundkörper und ein direkt auf den Grundkörper aufgeflochtenes, den Grundkörper in Umfangsrichtung umgebendes und als Radialstütze dienendes Fadenflechtwerk, wobei die äußere Schicht des Implantates zur Anwachshilfe stellenweise mit einem Geflecht mit strukturierter Oberfläche abgedeckt ist.

Ein Implantat dieser Art ist aus der DE 39 09 545 bekannt, in der ein Schaft einer Hüftgelenk-Endoprothese beschrieben ist. Der bekannte Schaft besteht aus einem inneren Kern, einer als Fadenflechtwerk ausgebildeten Zwischenschicht und einer spritzgegossenen, die Endform bildende Außenschicht, wobei diese drei Komponenten sich von dem distalen Ende bis zum proximalen Ende des Schaftes erstrecken.

Mit einem auf diese Weise zusammengesetzten Implantat soll eine Anpassung an die Flexibilität des Knochens erreicht werden. Die aus Polyätherätherketon bestehende Außenschicht eignet sich nicht für den Anwachsprozeß im menschlichen Knochenmaterial. Aus diesem Grund werden bei der bekannten Vorrichtung im Mittelbereich des Schaftes Metallfaserkissen vorgesehen, in die das Knochenmaterial und/oder ein Knochenzement eindringen soll. Bei einer derartigen Ausführung ist es zweifelhaft, ob ein in sich bewegliches Kissen als Zwischenschicht zur Verbindung zweier Komponenten geeignet ist.

Anstelle des Metallfaserkissens ist gemäß der EP 225 838 ein Drahtgeflecht als Anwachshilfe vorgesehen, das im Vorder- und Rückbereich des proximalen Endes eines Schaftimplantates mit diesem verklebt ist. Dieser Verbund könnte nach Einwachsen von Knochenmaterial ebenfalls durch die Belastungen gelöst werden, mit der Folge, daß das Implantat sich lockert.

Der Erfindung liegt die Aufgabe zugrunde, ein Knochenimplantat der eingangs genannten Art dahingehend zu entwickeln, daß eine gute Haftung des Implantates im Knochenmaterial durch entsprechenden Anwachsprozeß erreicht werden kann.

Die Aufgabe wird erfindungsgemäß durch die Merkmale des Anspruchs 1 gelöst.

Durch das Flechtwerk erhält das Implantat eine strukturierte Oberfläche, die gut für den erwünschten Anwachsprozeß des Knochenmaterials geeignet ist und den Vorteil hat, daß die Struktur fest mit dem Implantat verbunden ist und außerdem als Radialstütze für den im Einsatz stark beanspruchten Grundkörper dient.

Das Flechtwerk besteht vorzugsweise aus stark verdrallten Fäden, so daß deren runder Querschnitt auch im Flechtwerk die Oberflächenstruktur verstärkend weitgehend erhalten bleibt.

Gemäß einer weiteren Ausgestaltung der Erfindung besteht das Flechtwerk aus einem erhöhten Faseranteil, der bis zu 70% einnehmen kann. Auf diese Weise wird erreicht, daß die durch die Fäden erreichbare Oberflächenstruktur sich durchsetzt, d. h., daß die Vertiefungen nicht mit Matrixmaterial gefüllt werden. Bei einem nur 30%-igen Anteil von Matrixmaterial kann erreicht werden, daß einerseits die Fäden in sich und mit dem Kern eine innige Verbindung eingehen und andererseits kein Überschuß an Matrixmaterial besteht. Der Grundkörper kann jede bewährte Ausgestaltung haben sowie aus bewährten Materialien bestehen.

Das Fadenflechtwerk wirkt sich aber besonders in Verbindung mit einem Grundkörper gut aus, der vorwiegend aus unidirektionalen, d. h. längs des Schaftes gerichteten Fasern besteht. Aufgrund der Querschnittsänderung enthält ein derartiger Schaft in inneren Bereichen einen als Verdrängerkörper wirkenden Kern. Ein derartiger Grundkörper hat die optimale Auslegung zur Aufnahme und Weiterleitung der auf einen Prothesenschaft einwirkenden Belastungen, insbesondere in Form von Druckbelastungen. Ergänzt lediglich durch das Flechtwerk ergibt sich eine Rundumoptimierung, weil das als Anwachshilfe dienende Flechtwerk gleichzeitig Radial- und Torsionskräfte aufzunehmen vermag. Ein derartiger Schaft ist außerdem einfach herzustellen und für Serienfertigung geeignet.

Bei einem derartigen Implantat bildet die Ummantelung den eigentlichen kraftaufnehmenden und -weiterleitenden Körper. Dazu erstrecken sich die längsgerichteten Fasern in ihrer Gesamtheit vom einen bis zum anderen Ende des Schaftes. Aufgrund der unregelmäßigen Geometrie des Schaftes ist ein innerer Kern erforderlich, der innerhalb der Ummantelung als Verdrängerkörper dient. Auf diese Weise werden die Fasern optimal genutzt, indem sämtliche, die Kräfte aufnehmenden Fasern an der Krafteinleitung in andere Bereiche des Schaftes beteiligt werden. Der Kraftschluß bleibt in der Ummantelung, d.h. an der Schaftoberfläche bzw. in der Umgebung des umliegenden Knochenmaterials im einplantierten Zustand. Damit werden die Kräfte über die Fasern direkt an die Knochenanwachszone oder gegebenenfalls an ein dazwischenliegendes Klebmaterial eingeleitet. Bei diesen Kräften handelt es sich vorwiegend um in Längsrichtung des Schaftes gerichtete Druckkräfte.

Gemäß einer weiteren Ausgestaltung der Erfindung ist die Ummantelung durch mindestens ein Fadenflechtwerk durchsetzt, womit eine Abhilfe für Biegekräfte geschaffen wird, die im Verlauf von Bewegungen im Schaft einwirken.

Mit den Fadenflechtwerken bietet sich eine optimale radiale Stütze für die unidirektionalen Fasern der Ummantelung. Die geflochtenen Fasern halten sich gegenseitig aufgrund ihrer Flechtführung stabil in ihren Ursprungspositionen, was insbesondere im konvexen Bereich des gebogenen Schaftes, der am stärksten beansprucht wird, wichtig ist. Die radial stützenden Fäden können daher nicht wie bei bekannten im Wickelverfahren aufgelegten schrägen oder in Umfangsrichtung gerichteten Fäden seitlich weggedrängt werden.

Mit dem inneren Flechtwerk wird zusätzlich einer seitlichen Verschiebung der unidirektionalen Fasern im konvexen Bereich entgegengewirkt, indem die unidirektionalen Fasern einerseits in zwei Bereiche aufgeteilt und andererseits die Kontaktfläche zwischen unidirektionalen Fasern und der strukturierten Oberfläche der Flechtwerke vergrößert wird, die die unidirektionalen Fasern in ihrer Ursprungslage zu verankern versuchen. Die beiden Flechtwerke bilden eine Art Doppeltorsionskasten, die in Verbindung mit den unidirektionalen Fasern die Steifigkeit des Schaftes gegenüber allen auftretenden Kräften optimal einstellen.

Nachdem die Strukturierung der Fäden im Flechtwerk einen strengen bzw. straffen Verlauf der unmittelbar angrenzenden unidirektionalen Fasern leicht stören, ist es vorteilhaft, daß der größte unidirektionale Faseranteil die innere Ummantelungslage bildet, während nur ein Restanteil von etwa 30% zwischen den beiden Flechtwerken zu liegen kommt. Bei einer größeren Anzahl von unidirektionalen Faserlagen kann der Anteil zwischen den beiden Faden flechtwerken bis maximal 50% angehoben werden.

Die Fasern des Fadenflechtwerkes haben vorzugsweise über die gesamte Schaftlänge etwa eine Orientierung von ± 45°.

Die Erfindung erstreckt sich auf ein Verfahren zur Herstellung eines Knochenimplantats, das durch die Maßnahmen des Anspruches 9 gekennzeichnet ist.

Der vorgefertigte Grundkörper bildet den eigentlichen, insbesondere axiale Druckkräfte aufnehmenden Schaft. Der Grundkörper ist ein Verbund-, insbesondere Faserverbundkörper oder ein hinsichtlich des Materials homogenes Bauteil, auf den bzw. auf das die Flechtfäden direkt aufgeflochten werden. Dadurch wird erreicht, daß die Fäden über das gesamte Bauteil trotz der unregelmäßigen Formgebung die gleiche Spannung erhalten. Durch das Schwenken des Grundkörpers während des Flechtvorganges wird die Orientierung der Fäden über den gesamten Schaft bestimmt. Dabei kann die Orientierung über die Schaftlänge entweder nach gewünschten Kriterien verändert oder durchweg gleichgehalten werden. Je nach gewünschter Orientierung wird die Schwenk- und Vorschubbewegung des Grundkörpers ausgelegt. Wichtig ist, daß dabei gleichzeitig die Fadenspannung, die durch die Bremswirkung der Fadenversorgungseinrichtung (z.B. Klöppel) und der Vorschubgeschwindigkeit des Grundkörpers bestimmt wird, kontrollierbar beeinflußt werden kann. Dieses gilt sowohl für das bzw. die inneren Flechtwerke als auch für das äußere Flechtwerk.

Bei einem Faserverbundschaft dient das äußere Flechtwerk nicht nur als Anwachshilfe, sondern auch als, wie bei gegebenenfalls vorgesehenen inneren Flechtwerken, Torsionskasten bzw. radiale Stütze für im Grundkörper enthaltene Faserlagen. Die Kombination von inneren, längsgerichteten (unidirektionalen) Fasern mit einem äußeren Fadenflechtwerk bildet einen fertigungstechnisch sehr einfach herstellbaren Schaft, der optimale mechanische Eigenschaften zur Verwendung für eine Hüftgelenk-Endoprothese hat, was durch Verwendung von Kohlenstoffasern (C-Fasern) weiter unterstützt werden kann. C-Fasern haben nicht nur günstige Festigkeitseigenschaften, sondern sie sind auch körperverträglich.

Verfahrenstechnisch lassen sich derartige Schäfte serienmäßig unter Verwendung eines Stützkernes mit einer Flechtmaschine herstellen, in der die unidirektionalen Fasern gleichzeitig mit den Flechtfaden auf den Stützkern aufgebracht werden.

Für den Flechtvorgang der äußeren Flechtlage werden vorzugsweise verdrallte Fäden verwendet, die bis zu 30 mal pro Meter verdrallt sind. Derartige Fäden erhalten ihren runden Querschnitt weitgehend im Flechtwerk, auch wenn dieses mit unter höherer Spannung gehaltenen Fäden geflochten wurde.

Weiterhin wird vorgeschlagen, im Fadenflechtwerk einen geringen Matrixanteil vorzusehen. Dieser wird mit stark verdünntem (z .B. mit Aceton) Matrixmaterial erreicht.

Gemäß einem weiteren Vorschlag der Erfindung wird der Grundkörper vor Aufbringung des Flechtwerkes mit Harz überzogen, das aufgestrichen, durch Tauchtränkung oder ähnlichem aufgebracht wird. Auch hier kann verdünntes Harz verwendet werden. Damit kann der Harzanteil in den Fäden lediglich zur Verbindung der Einzelfasern eines Fadens auf ein Mindestmaß gebracht werden, während die Verbindung mit dem Grundkörper durch das auf den Grundkörper aufgetragene Matrixmaterial erfolgt. Damit wird kontrollierbar verhindert, daß ein Überschuß an Matrixmaterial die Oberfläche des Flechtwerkes ebnet.

Wenn man in einem Bereich eines Implantates das Anwachsen des Knochens verhindern will, dann wird dieser Bereich gemäß einer weiteren Ausgestaltung der Erfindung einer Wärme/Druckbehandlung unterzogen, bei der die Oberfläche des betreffenden Bereiches durch Abformung abgeflacht wird.

Bei einem Schaft für ein Hüftgelenk-Implantat beispielsweise wird das distale Ende des Schaftes, das in den Femurknochen eingeführt wird, vorzugsweise auf diese Weise behandelt, um den Anwachsbereich auf das obere Ende des in den Femur eingeführten Schaftes zu beschränken und die untere Spitze des Schaftes freizuhalten, insbesondere dann, wenn der Schaft nicht die Biegeelastizität des Knochens erreicht.

Es ist aber auch möglich, in derartigen Fällen das Implantat nur in den Bereichen mit einem Flechtwerk zu versehen, die mit dem Knochenmaterial zusammenwachsen sollen.

Bei Anwendung von biokompatiblen Fasern, wie z.B. C-Fasern und einem weniger biokompatiblen Matrixmaterial, ist es ohne weiteres möglich, mittels einer mechanischen Nachbehandlung die Fasern an der Oberfläche freizulegen, ohne sie zu beschädigen. Das kann beispielsweise durch Überbürsten mit Spezialbürsten oder durch Sand-, Korund-Bestrahlung geschehen.

Die Erfindung wird nachfolgend anhand von in der Zeichnung schematisch dargestellten Ausführungsbeispielen näher beschrieben. Es zeigen:
- Fig. 1: eine Anlage zum Durchführen des Verfahrens im Querschnitt,
- Fig. 2: den Faserverlauf an einem Bauteil,
- Fig. 3: ein erstes Ausführungsbeispiel,
- Fig. 4 und 5: je einen Querschnitt des Faserflechtwerkes,
- Fig. 6: ein zweites Ausführungsbeispiel,
- Fig. 7: einen Querschnitt aus Fig. 6

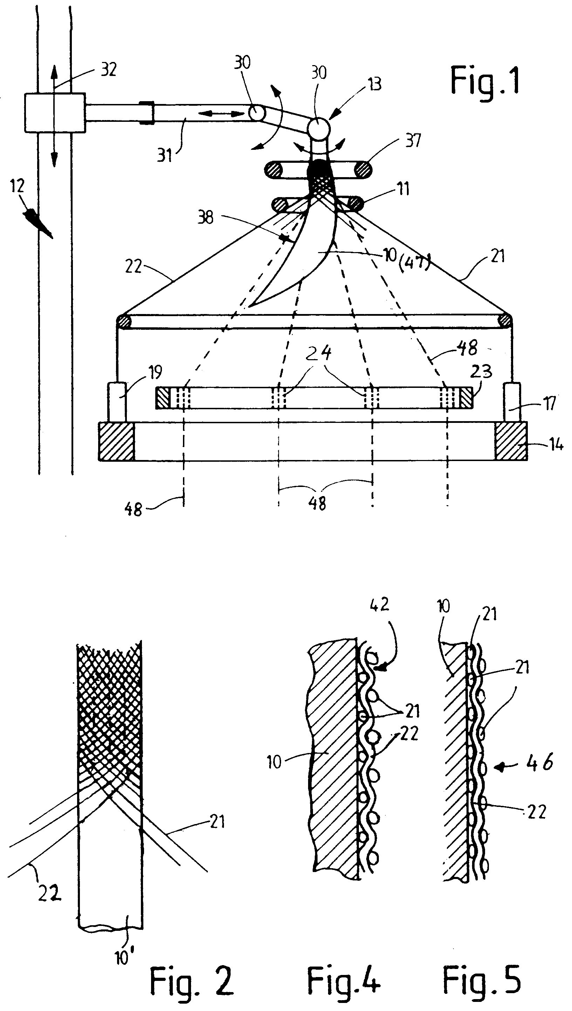

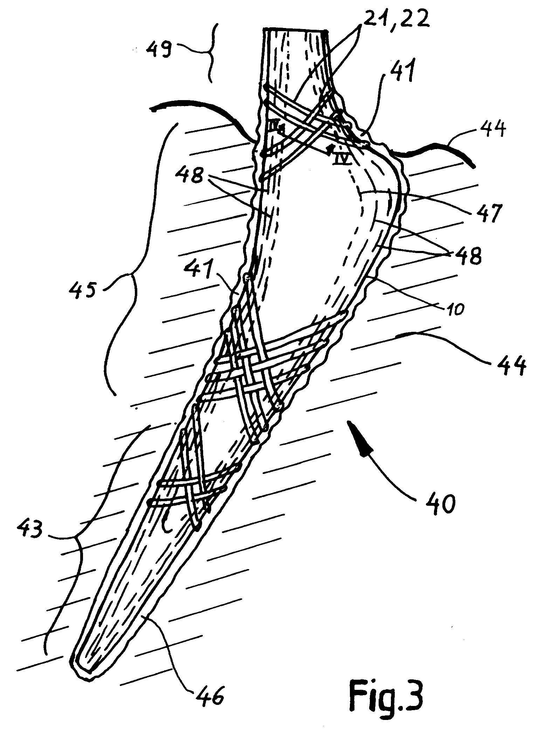

In Fig. 1 ist eine Anordnung zur Herstellung eines Prothesenschaftes gezeigt, der zunächst aus einem vorgefertigten Grundkörper 10 besteht. Der Grundkörper wird durch ein Fadenauge 11 einer konventionellen Flechtmaschine 12 mittels eines Greifers 13 durchgezogen. Die Flechtmaschine 12 besteht im wesentlichen aus einem Führungsring 14, auf dem zwei nicht dargestellte sinusförmige Führungsschienen für eine bestimmte Anzahl von Klöppel 17 bzw. 19 vorgesehen sind, die in zwei Gruppen aufgeteilt gegenläufig in den Führungsschienen bewegt werden.

Zu Beginn des Flechtvorganges werden sämtliche Enden der Fäden 21, 22 mit dem Greifer 13 verbunden, der gleichzeitig den Kern trägt und ihn mit einer vorgegebenen Geschwindigkeit vom Fadenauge 11 wegbewegt. Für gekrümmte oder unregelmäßige Implantate 40, wie es in dem in Fig. 3 gezeigten Prothesenschaft der Fall ist, ist der Greifer 13 mit Gelenken 30, Teleskopeinrichtungen 31 oder dergleichen ausgerüstet, um zusätzlich zur Vorschubbewegung 32 auch noch Schwenkbewegungen durchführen zu können. Damit kann das Bauteil jeweils so gerichtet werden, daß die Fäden 21, 22 die gewünschte Orientierung im jeweiligen Implantatbereich erhalten.

Aufgrund der Bewegung der Klöppel 17 bzw. 19 in den Führungsschienen beschreiben die einzelnen Flechtfäden 21 bzw. 22 im Verlauf des Flechtprozesses gegenläufige Schraubenlinien, wie sie in Fig. 2 an einem einfachen zylindrischen Bauteil 10' gezeigt sind, wobei jeder einzelne Faden 22 abwechselnd unter und über andere Fäden 21 gelegt wird, wie Fig. 4 zeigt.

Die Steigung der umwickelten Flechtfäden bzw. deren Orientierung am fertigen Bauteil wird durch die Geschwindigkeit und Lage bestimmt, mit der der Grundkörper 10 durch das Fadenauge 11 geführt wird, wobei die geometrischen Verhältnisse der Flechtmaschine 12 und der jeweilige Querschnitt des Grundkörpers 10 Berücksichtigung finden. Durch Programmierung des Greifer-Bewegungsmechanismus kann eine Veränderung der Vorschubgeschwindigkeit sowie der Schwenklage entsprechend der Geometrie bzw. dem Querschnitt des Grundkörpers 10 automatisiert werden, derart, daß der Grundkörper in jedem Bereich die Orientierung der Flechtfäden 21, 22 erhält, die im jeweiligen Bereich gewünscht wird.

Ein weiterer Parameter ist die Zahl der Fäden 21, 22, diese richtet sich nach der Dimension des herzustellenden Bauteiles sowie der gewünschten Flechtwerkdichte bei vorgegebener Fadenorientierung. Für einen Schaft für Hüftgelenk-Implantate werden für eine etwa 45°-Lage 20 bis 40 Fadenstränge 21, 22 verwendet.

Für die Umflechtung eines Grundkörpers 10 wird dieser mit Matrixmaterial durch Tauchtränken, Bestreichen und ähnliche Verfahren überzogen und zusammen mit den Fadenenden am Greifer 13 befestigt. Der Greifer 13 wird mit einer vorgegebenen und unter Umständen während des Flechtprozesses sich verändernden Geschwindigkeit bewegt, wobei dessen Winkellage sich entsprechend der Krümmung des Grundkörpers 10 verändert. Die gewünschte Fadenspannung (beispielsweise 350 g) erfolgt durch Bremswirkung der Klöppel 17, 19.

Die Fäden 21, 22 werden jeweils durch nicht dargestellte Tränkvorrichtungen geleitet, in denen ein stark, beispielsweise mit Aceton verdünntes Matrixmaterial enthalten ist.

Der Verdünnungsgrad wird so gewählt, daß der am fertigen Bauteil nach dem Verflüchtigen des Verdünnungsmittels verbleibende Harzanteil gerade ausreicht, um die Einzelfasern eines jeweiligen Fadens, die Fäden miteinander und mit dem Grundkörper zu verbinden, so daß kein nennenswerter Harzüberschuß verbleibt. Die Verbindung der Fäden mit dem Grundkörper 10 wird durch das auf den Grundkörper 10 aufgetragene Matrixmaterial unterstützt.

Bei diesem Verfahren erhält die Oberfläche des fertigen Implantats 40 die Struktur des Fadengeflechtes, das mit einer sehr dünnen gleichmäßigen Matrixmaterialschicht überzogen ist. Die Strukturierung kann durch Anwendung von stark verdrallten Fäden 21, 22 betont werden. Dazu werden entsprechend verdrallte Faden auf die Klöppel 17, 19 aufgewickelt, wobei die Verdrallrichtung bei den beiden Klöppelgruppen 17 und 19 umgekehrt sein muß, damit die Verdrallung beim Flechtprozeß nicht wieder gelöst wird. Die Verdrallstärke wird vorzugsweise empirisch ermittelt. Bei C-Fasern hat sich eine Verdrallung bis zu 30 mal/m als günstig erwiesen. Die in sich verdrallten Fasern 21, 22 behalten im Flechtwerk nahezu ihren runden Querschnitt, wodurch die Strukturierung an der Oberfläche verstärkt wird, wie in Fig. 4 gezeigt ist.

Durch die Verwendung von geringem Anteil von Matrixmaterial sind die Fäden 21, 22 lediglich mit einem dünnen Matrixfilm überzogen, dessen Dicke sich nahezu gleichmäßig, d. h. ohne in den Vertiefungen 42 sich zu verstärken, über die Oberfläche erstreckt. Das heißt, die dem Flechtwerk eigene Strukturierung bleibt erhalten. Dadurch ist es darüber hinaus möglich, durch eine kurze mechanische Nachbehandlung den Matrixfilm an der gesamten Oberfläche des Flechtwerkes 41, ohne die Fasern zerstören zu müssen, zu entfernen und die Fasern 21, 22 freizulegen. Dieses ist insbesondere für die Fälle von Vorteil, bei denen die Biokompatibilität der verwendeten Faser 21, 22 besser als die des Matrixmaterials ist.

Das vorstehend beschriebene Verfahren läßt sich zur serienmäßigen Fertigung von mit Flechtwerk überzogenen Implantaten anwenden. Das direkt auf den Grundkörper aufgeflochtene Flechtwerk ermöglicht zudem die Ausgestaltung von sehr einfach herstellbaren Implantaten mit optimalen mechanischen Eigenschaften. Das Faserflechtwerk, das als Anwachshilfe dient, übernimmt bei entsprechender Orientierung von ca. ± 45° und Spannung von hochfesten Fasern, wie C-Fasern, Torsions- und Radialbelastungen auf. Mit einer inneren Schicht 0°-orientierten Fasern vervollständigt sich das Belastungsbild, indem diese unidirektionalen, längs des Schaftes gerichteten Fasern die Druck- und Zugbelastungen aufnehmen und radial durch das Flechtwerk gestützt werden. Aufgrund der unterschiedlichen Querschnitte des Schaftes können die längsgerichteten Fasern, deren Gesamtquerschnitt durch das dünnere Ende (obere oder untere) des Schaftes bestimmt wird, den Schaft im dickeren Mittelbereich nicht ausfüllen. Hier dient ein Kern, vorzugsweise aus Kurzfasern, als Verdrängerkörper. Es hat sich gezeigt, daß ein derartig aufgebauter Schaft die Belastungen im implantierten Gebrauch ausgezeichnet und auf Dauer aufnimmt und in den umgebenden Knochen einleitet.

Die Herstellung des Implantats kann auf zwei Verfahrensschritte eingeschränkt werden, wobei in einer ersten Stufe der Kern je nach Material entsprechend gefertigt wird. Bei einem Faserverbund-Kern wird das Preßverfahren zu wählen sein. In der zweiten Stufe wird der Verbund des Kernes mit den längsgerichteten Fasern sowie mit den Flechtfäden geschaffen. Hierzu wird die Flechtmaschine 12 mit einem zusätzlichen Fadenführungsring 23 ausgestattet, durch dessen Fadenaugen 24 Fasern 48 für die unidirektionalen Lagen durchgeführt werden. Diese Fasern 48 werden zusammen mit den Flechtfäden 21, 22 in einem Arbeitsgang auf den Kern 47 aufgebracht. In diesem Fall nimmt der Kern 47 die Stelle des Grundkörpers 10 ein.

In Fig. 3 ist ein fertiggestellter Schaft 40 für eine Hüftgelenk-Endoprothese gezeigt, der aus dem inneren Kern 47, den unidirektionalen Fasern 48 und einem damit fest verbundenen Flechtwerkschlauch 41 besteht. Der Kern 47 füllt den Innenraum im Bereich 45 des größeren Querschnitts des Schaftes 40 aus. Das distale Ende 43 besteht vollständig aus unidirektionalen Fasern 48 und dem umgebenden Flechtwerk.

Bei einem Hüftgelenkschaft ist es vorteilhaft, wenn das distale Ende 43 desselben im Knochenmaterial 44 frei bleibt, um unterschiedliche Biegesteifigkeiten zwischen Knochen 44 und Implantat 40 ausgleichen zu können. In diesem Fall wird die Oberfläche des distale Endes 43 vorzugsweise unstrukturiert ausgestaltet. Dieses kann bei einem Grundkörper 10 aus homogenem Material beispielsweise dadurch geschehen, daß lediglich in der Anwachszone 45 ein Flechtwerk 41 auf das Implantat aufgebracht wird.

Bei einem Faserverbund-Schaft, insbesondere einem aus Kern 48 und unidirektionalen Fasern 48 bestehenden Grundkörper, wird der gesamte Grundkörper 10 mit dem gleichzeitig als Torsionskasten dienenden Flechtwerkschlauch 41 zu überziehen sein. Eine möglichst glatte Oberfläche am proximalen Ende 43 wird dann dadurch erreicht, daß das fertiggeflochtene Implantat 40 anschließend an dem betreffenden Ende 43 einer Wärme/Druckbehandlung unterzogen wird, bei der die Fäden 21, 22, wie in Fig. 5 gezeigt, abgeflacht werden und damit das Flechtwerk an dieser Stelle 46 ihre Oberflächenstruktur nahezu verliert. Das gleiche erfolgt am anderen Ende 49, auf das eine nicht dargestellte Gelenkkugel aufgesetzt wird. Der gesamte Faserverbund-Schaft kann aus einem Fasermaterial und einem Matrixmaterial bestehen.

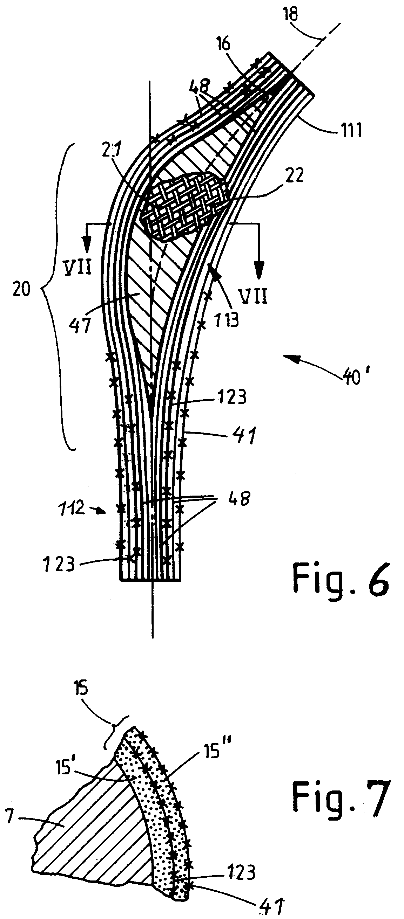

In Fig. 6 ist ein Längsschnitt eines Schaftes 40' für Hüftgelenk-Endoprothesen dargestellt, der ein annähernd zylindrisches oberes Ende 111 für die Aufnahme eines nicht dargestellten Gelenkkopfes, ein unteres Ende 112 und einen im Querschnitt erweiterten, geknickten Mittelbereich 113 hat. Der Schaft 40' wird im wesentlichen aus unidirektionalen Fasern 48 gebildet, die in Längsrichtung des Schaftes 40' sich von dem einen Ende 111 bis zum anderen Ende 112 des Schaftes 40' erstrecken.

Aufgrund der Querschnittserweiterung im mittleren Bereich 113 und des größeren Querschnittes des oberen Endes 111 bilden die unidirektionalen Fasern 48 im Mittelbereich eine Ummantelung 15, während sie am oberen Ende 111 einen dünnen Kanal 16 freilassen. Der Hohlraum zwischen den unidirektionalen Fasern 48 ist mit einem Kern 47 lückenlos ausgefüllt, der nach innen gerichtete Radialkräfte aufzunehmen vermag.

Um ebenfalls nach außen gerichtete Radialkräfte, die im Gebrauch des Schaftes insbesondere im konvexen Bogenbereich 20 auftreten werden, aufnehmen zu können, wird der Schaft 40' zusätzlich zum äußeren Flechtwerk 41 mit mindestens einem Flechtwerk 113 ergänzt. Die innere Fadenflechtwerklage 123 befindet sich zwischen den unidirektionalen Faserlagen, und zwar derart, daß die Ummantelung 15 in zwei Lagen bzw. Lagengruppen 15' und 15'' getrennt werden. Dieses zweite, innenliegende Flechtwerk 123 unterstützt die Wirkung des äußeren Flechtwerkes 41. Wie in Fig. 6 in der ausschnittsweise dargestellten Draufsicht des äußeren Flechtwerkes 41 erkennbar ist, werden die Fäden 21 einer Richtung abwechselnd unter den über die Fäden 22 der Querrichtung geführt, wobei sich zu beiden Seiten eines Flechtwerkes 41 bzw. 123 eine strukturierte Oberfläche ergibt. Diese Strukturierung erlaubt gleichzeitig eine tangentiale Verankerung, zumindest der an die Flechtwerke angrenzenden unidirektionalen Fasern 48. Auch diese Wirkung wird durch die Aufteilung des Fadenflechtwerkes in zwei nicht unmittelbar aneinanderliegenden Flechtwerklagen 41, 123 verstärkt.

Auf der anderen Seite jedoch wird der Faserverlauf der unidirektionalen Fasern im Grenzbereich der Flechtwerke 41, 123 durch die Oberflächenstrukturierung beeinflußt, so daß deren Spannung leicht abfällt. Um diesen Effekt möglichst gering zu halten, wird das innere Flechtwerk 123 in radialer Richtung so angeordnet, daß im inneren, d.h. am Kern 47 angrenzenden Bereich 15', der größere Anteil der unidirektionalen Fasern 48 liegt. Dieser Anteil bewegt sich je nach Anzahl der unidirektionalen Faserlagen 48 zwischen 50 bis 90% der gesamten unidirektionalen Fasern.

## Patentansprüche

1. Knochenimplantat, enthaltend einen Grundkörper (10) und ein direkt auf den Grundkörper aufgeflochtenes, den Grundkörper in umfangsrichtung umgebendes und als Radialstütze dienendes Fadenflechtwerk (41), dadurch gekennzeichnet, daß das Fadenflechtwerk (41) die äußere Schicht des Implantats (40) bildet und eine strukturierte Oberfläche (21, 22) hat.

2. Implantat nach Anspruch 1, dadurch gekennzeichnet, daß das Flechtwerk (41) aus stark verdrallten Fäden (21, 22) hergestellt ist, die im Flechtwerk ihren runden Querschnitt (Fig. 4) weitgehend beibehalten, daß ein erhöhter Faseranteil, vorzugsweise bis 70%, vorgesehen ist und daß die Oberfläche des Fadenflechtwerks (41) aus Fadenmaterial besteht.

3. Implantat nach einem der vorhergehenden Ansprüche, dadurch gekennzeichnet, daß der Grundkörper (10) aus einem Kern (47) und einer Ummantelung aus unidirektionalen Fasern (48) besteht, die mindestens teilweise vom Fadenflechtwerk (41) umgeben sind.

4. Implantat nach Anspruch 3, dadurch gekennzeichnet, daß der Kern (47) des Grundkörpers (10, 10') aus Faserverbundmaterial besteht.

5. Implantat nach Anspruch 3 oder 4, dadurch gekennzeichnet, daß die Ummantelung (15) mit mindestens einem Fadenflechtwerk (123) durchsetzt ist.

6. Implantat nach Anspruch 5, dadurch gekennzeichnet, daß 50 bis 90%, vorzugsweise etwa 70% der unidirektionalen Fasern (48) der Ummantelung (15') sich unterhalb des inneren Flechtwerkes (123) und die übrigen 50 bis 10% bzw. etwa 30% sich zwischen dem inneren und dem äußeren Flechtwerk (123 bzw. 41) befinden und daß die Fäden (21, 22) der Flechtwerke (41, 123) über den gesamten Schaft (40') etwa ± 45° zur Schaftachse (18) gerichtet sind.

7. Implantat nach Anspruch 3 oder 4, dadurch gekennzeichnet, daß die Fasern (48, 21, 22) der unidirektionalen Lagen, der Flechtwerke (41) und gegebenenfalls des Kernes (47) aus dem gleichen Material bestehen und daß die Matrix der unidirektionalen Lagen, der flechtwerke (41) und des Kernes (47) aus dem gleichen Material bestehen.

8. Verfahren zur Herstellung eines Knochenimplantats nach Anspruch 1, dadurch gekennzeichnet, daß das Flechtwerk (41) mittels einer Flechtmaschine (12) unter Einhaltung einer gleichmäßigen Spannung der Fäden (21, 22) hergestellt wird, und daß der Grundkörper (10) während des Flechtvorganges zur Einbringung der gewünschten Orientierung der Flechtfäden (21, 22) geschwenkt wird.

9. Verfahren nach Anspruch 8, dadurch gekennzeichnet, daß die Flechtmaschine (12) mit bis zu 30 mal/m verdrallten Fäden (21, 22) bestückt wird und daß die Flechtfäden (21, 22) für das äußere Flechtwerk (41) mit stark verdünntem Matrixmaterial getränkt werden.

10. Verfahren nach einem der Ansprüche 8 oder 9, dadurch gekennzeichnet, daß der Grundkörper (10, 10') vor Aufbringung des Flechtwerkes (41) mit Harz überzogen wird.

11. Verfahren nach einem der Ansprüche 8 bis 10, dadurch gekennzeichnet, daß bei der Herstellung eines Schaftes (40) für ein Hüftgelenkimplantat der Schaft nach Aufbringung des äußeren Flechtwerkes (41) einer Wärmebehandlung unterzogen wird, bei der auf das distale Ende (43) des Schaftes zur Abflachung der Oberflächenstruktur (46) Druck ausgeübt wird.

12. Verfahren nach einem der Ansprüche 5 bis 8, dadurch gekennzeichnet, daß bei der Herstellung eines Schaftes (40) für ein Hüftgelenkimplantat der Schaft nur im Bereich (45) nahe dem proximalen Ende mit einem äußeren Flechtwerk (41) versehen wird.

13. Verfahren nach einem der vorhergehenden Ansprüche, dadurch gekennzeichnet, daß die Oberfläche des äußeren Flechtwerkes (41) nach Fertigstellung des Implantats (40) von Restmatrixmaterial befreit wird.

## Claims

1. Bone implant, containing a basic body (10) and a thread braiding (41) braided directly onto the basic body, surrounding the basic body in the peripheral direction and serving as a radial support, characterized in that the thread braiding (41) forms the outer layer of the implant (40) and has a textured surface (21, 22).

2. Implant according to Claim 1, characterized in that the braiding (41) is produced from highly twisted threads (21, 22) which in the braiding substantially retain their round cross-section (Fig. 4), in that a high proportion of fibres, preferably up to 70%, is provided, and in that the surface of the thread braiding (41) comprises thread material.

3. Implant according to one of the preceding claims, characterized in that the basic body (10) comprises a core (47) and a casing of unidirectional fibres (48) which are at least partially surrounded by the thread braiding (41).

4. Implant according to Claim 3, characterized in that the core (47) of the basic body (10, 10') comprises composite fibre material.

5. Implant according to Claim 3 or 4, characterized in that at least one thread braiding (123) passes through the casing (15).

6. Implant according to Claim 5, characterized in that 50 to 90%, preferably approximately 70%, of the unidirectional fibres (48) of the casing (15') are below the inner braiding (123) and the other 50 to 10% or approximately 30% are between the inner and outer braiding (123 and 41 respectively), and in that the threads (21, 22) of the braidings (41, 123) are directed over the entire shaft (40') at approximately ± 45° to the shaft axis (18).

7. Implant according to Claim 3 or 4, characterized in that the fibres (48, 21, 22) of the unidirectional layers, of the braidings (41) and where appropriate of the core (47) are of the same material, and in that the matrix of the unidirectional layers, of the braidings (41) and of the core (47) are of the same material.

8. Process for producing a bone implant according to Claim 1, characterized in that the braiding (41) is produced by means of a braiding machine (12) maintaining a uniform tension of the threads (21, 22), and in that the basic body (10) is pivoted during the braiding process to bring the braiding threads (21, 22) into the desired orientation.

9. Process according to Claim 8, characterized in that the braiding machine (12) is equipped with threads (21, 22) twisted up to 30 times/m, and in that the braiding threads (21, 22) for the outer braiding (41) are impregnated with highly dilute matrix material.

10. Process according to one of Claims 8 or 9, characterized in that the basic body (10, 10') is coated with resin before the braiding (41) is applied.

11. Process according to one of Claims 8 to 10, characterized in that, in producing a shaft (40) for a hip joint implant, the shaft is subjected, after the outer braiding (41) has been applied, to a heat treatment during which pressure is exerted on the distal end (43) of the shaft to flatten the surface texture (46).

12. Process according to one of Claims 5 to 8, characterized in that, in producing a shaft (40) for a hip joint implant, the shaft is provided with an outer braiding (41) only in the region (45) close to the proximal end.

13. Process according to one of the preceding claims, characterized in that the surface of the outer braiding (41) is freed from remaining matrix material on completion of the implant (40).

## Revendications

1. Implant osseux composé d'un corps de base (10) et d'un tissu tressé de fils réalisé directement sur le corps de base et entourant celui-ci en servant d'appui radial, caractérisé en ce que le tissu tressé de fils (41) forme la couche extérieure de l'implant (40) et présente une surface structurée (21, 22).

2. Implant osseux selon la revendication 1, caractérisé en ce que le tissu tressé (41) est formé de fils (21, 22) à forte torsion, conservant dans une très large mesure leur section circulaire dans le tissu tressé (figure 4) avec une fraction augmentée de fibres en particulier jusqu'à 70 %, et la surface du tissu tressé de fils (41) est en un matériau en fibres.

3. Implant selon l'une des revendications précédentes, caractérisé en ce que le corps de base (10) se compose d'un noyau (47) et d'une enveloppe en fibres (48) unidirectionnelles qui sont au moins partiellement entourées par le tissu tressé de fils (41).

4. Implant selon la revendication 3, caractérisé en ce que le noyau (47) du corps de base (10, 10') est en un matériau en fibres composite.

5. Implant selon la revendication 3 ou 4, caractérisé en ce que l'enveloppe (15) est traversée par au moins un tissu tressé de fils (123).

6. Implant selon la revendication 5, caractérisé en ce qu'entre 50 et 90 % et de préférence environ 70 % des fibres unidirectionnelles (48) de l'enveloppe (15') se trouvent sous le tissu tressé intérieur (123) et les autres 50 à 10 % ou environ 30 % se trouvent entre le tissu intérieur et le tissu extérieur (123, 41), et les fils (21, 22) des tissus tressés (41, 123) sont inclinés environ à ± 45° par rapport à l'axe (18) de l'ensemble du corps (40').

7. Implant selon la revendication 3 ou 4, caractérisé en ce que les fibres (48, 21, 22) des nappes unidirectionnelles du tissu tressé (41) et le cas échéant du noyau (47) sont formées du même matériau et la matrice des nappes unidirectionnelles du tissu tressé (41) et du noyau (47) est réalisée dans la même matière.

8. Procédé de fabrication d'un implant osseux selon la revendication 1, caractérisé en ce que le tissu tressé (41) est réalisé à l'aide d'une machine à tresser (12) en conférant aux fils (21, 22) une tension régulière et le corps de base (10) est basculé pendant l'opération de tressage pour donner l'orientation souhaitée aux fils tressés (21, 22).

9. Procédé selon la revendication 8, caractérisé en ce que la machine à tresser (12) est alimentée en fils (21, 22) présentant une torsion allant jusqu'à 30 fois/m et les fils (21, 22) du tissu tressé extérieur (41) sont imprégnés d'un matériau de matrice fortement dilué.

10. Procédé selon l'une des revendications 8 ou 9, caractérisé en ce que le corps de base (10, 10') est revêtu de résine avant la mise en place du tissu tressé (41).

11. Procédé selon l'une des revendications 8 à 10, caractérisé en ce que lors de la réalisation d'un corps (40) pour un implant de hanche, le corps est soumis à un traitement thermique après application du tissu tressé extérieur (41) sur lequel est exercée la pression au niveau de l'extrémité distale (43) du corps pour aplatir la structure de la surface (46).

12. Procédé selon l'une des revendications 5 à 8, caractérisé en ce que pour la réalisation d'un corps (40) pour un implant de hanche, on munit le corps d'un tissu tressé extérieur (41) uniquement dans la zone (45) proche de l'extrémité proximale.

13. Procédé selon l'une des revendications précédentes, caractérisé en ce que la surface du tissu tressé extérieur (41) est dégagée de la matière résiduelle de la matrice après la fabrication de l'implant (40).
